# EUROPEAN PATENT SPECIFICATION

(11) **EP 3 813 749 B1**
(45) Date of publication and mention of the grant of the patent: **13.08.2025**
(21) Application number: 19740312.4
(22) Date of filing: 26.06.2019
(51) Int. Cl.: A61F 13/00

(54) **A DRESSING SYSTEM**
VERBANDSYSTEM
SYSTÈME DE PANSEMENT

(30) Priority: 27.06.2018 GB 201810531
(43) Date of publication of application: 05.05.2021
(73) Proprietor: University College Cork - National University of Ireland, Cork, Cork City (IE)
(72) Inventor: O'MAHONY, Conor, Cork, P17 WK40, Oysterhaven (IE); O'CALLAGHAN, Suzanne, Cork, T12 F9WT, Carrigrohane (IE)
(74) Representative: Purdylucey Intellectual Property
(86) International application number: PCT/EP2019/066975
(87) International publication number: WO 2020/002416

(56) References cited:
- EP-A1- 3 078 360
- WO-A1-2014/161036
- US-A1- 2009 227 969
- US-A1- 2017 304 510
- US-B2- 7 699 823

## Description

### Field

This disclosure relates to a wound dressing system having an integral sensor for monitoring and treating a wound.

### Background

A major requirement for effective wound care management is the need to monitor and change wound dressings to provide optimal conditions for tissue healing. Most known methods of wound monitoring and care require manual and/or subjective analysis of a range of parameters such as wound temperature and wound dressing saturation/age which usually involves disturbing the patient and/or removing the dressing for inspection. For example, it is recognized that wound temperature is a quantitative measurement that has the potential to assist in assessing and diagnosing chronic deep wound and surrounding skin infection. Indeed, studies have shown that incorporating quantitative skin temperature measurement into routine wound assessment provides a timely and reliable method for a clinician to quantify the heat associated with deep wound and surrounding skin infection (e.g. elevated local temperatures above 37°C are an indication of wound infection) and to monitor ongoing wound status.

Similarly, it is known that, as healing occurs, the amount of exudate produced usually decreases. The volume of exudate is related to the surface area of the wound and large wounds often produce higher volumes of exudate. However, although a moist wound environment is necessary for optimal wound healing, over- or under-production of exudate may adversely affect healing. Any factor that increases capillary leakage or predisposes a patient to the development of tissue oedema (e.g. inflammation, bacterial contamination or limb dependency) may boost exudate production while low exudate production may indicate a systemic problem (e.g. dehydration, hypovolemic shock and microangiopathy) or may be a feature of ischaemic ulcers. Accordingly, it is essential to accurately determine and evaluate the factors contributing to the production of too much or too little exudate.

However, currently, measurement and assessment of the aforementioned and other wound parameters are generally performed manually using subjective visual inspections and/or equipment such as thermometers or moisture meters resulting in suboptimal dressing changing frequency, clinical timewasting, patient discomfort, increased dressing cost and increased wound healing time.

Accordingly, various attempts have been made to develop smart dressings in which sensors such as a moisture sensor and an associated electronic module communicable with a clinician have been incorporated into the dressings. However, the placement of the sensor and module, as well as the construction of the dressing itself, can negatively impact on the performance of the smart dressing. More particularly, diffusion of wound exudate in a typical dressing takes place initially by vertical diffusion from a wound, through the dressing layers as far as a super-absorbent fibre (SAF) layer where fast lateral diffusion then takes place and the surface of this layer, which is generally in contact with the (moisture) sensor, gets rapidly wet. As a result, moisture is wicked across the dressing very rapidly into contact with the (moisture) sensor which is then activated (i.e. shows a sudden and significant change in measurand) and saturates earlier than desired for wound monitoring purposes i.e. before 50% of the dressing capacity is reached. As a result, it is not possible to measure or map the progress of the wound exudate through the dressing. The measurand can be, for example resistance, impedance, capacitance and/or optical properties.

It has also been found that dressing performance can be compromised by the presence of the electronic module and associated sensor in the dressing e.g. typically electronic modules are placed over the absorbent layers giving rise to pressure being generated by the electronic module on the absorbent layers in the dressing which can change the diffusion/wicking properties of the dressing so that the dressing does not perform as originally intended. Importantly, the placement of the electronic modules over the absorbent layers can also severely compromise the breathability of the dressings which is highly undesirable resulting in negative consequences for the healing process.

PCT Patent Specification No. WO 2017/195038 describes a dressing system for a wound comprising various sensors and an absorbent pad. However, the sensor(s) of the dressing system are not moisture sensors adapted to measure the progress of wound exudate across the dressing. Similarly, PCT Patent Specification No. WO 2017/129194 describes a dressing having a matrix of temperature sensors unsuitable for mapping wound exudate in the dressing, while US Patent Specification No. 2014/928927 describes a separate sensing device for attachment to a dressing in which the non-integral external sensing device has a plurality of separate moisture sensors which are arranged in such a way that progress of exudate across the dressing cannot be mapped. US 7699823 relates to a negative pressure wound dressing having a sealed, negative pressure device intended for drawing wound fluids into a vacuum reservoir. US 2017/0304510 discloses a reduced pressure wound dressing with saturation sensors that generate a signal based on exposure to liquid. WO 2014/161036. WO 2014/161036,relates to stimulus responsive substrates and uses of the substrates but fails to disclose a configuration of moisture sensors.

It is therefore an object to provide an improved dressing system to overcome at least one of the above mentioned problems

### Summary

According to a first aspect of the invention there is provided a dressing system for a wound comprising:
an absorbent pad;
at least one sensor for detecting wound data, and
an electronic module communicable with the sensor, wherein the sensor comprises a moisture sensor array provided on or in the absorbent pad adapted to map the progress of exudate in the absorbent pad, and wherein the moisture sensor array comprises a plurality of moisture sensors radially offset with respect to the centre of the absorbent pad including a peripheral moisture sensor located towards a periphery of the absorbent pad.

Preferably, the peripheral sensor is located beyond the periphery of the absorbent pad.

Preferably, the dressing system further comprises a diffusion barrier to delay diffusion of wound exudate towards the sensor.

Preferably, the diffusion barrier comprises a delay channel defined in the absorbent pad.

Preferably, the absorbent pad comprises a first superabsorbent layer and the diffusion barrier comprises a second superabsorbent layer between the sensor and the first superabsorbent layer, and wherein the first and second superabsorbent layers comprise superabsorbent fibre layers.

Preferably, the moisture sensors of the moisture sensor array are radially offset with respect to the centre of the absorbent pad, to delay activation of the moisture sensors by diffusing exudate until after about 50% of the dressing system capacity is reached.

Preferably, exudate progress across the dressing is measurable to provide a time-dependent indication of saturation levels of the dressing.

Preferably, the sensor further comprises one of: a pH sensor, a pressure sensor, a bacterial sensor, a temperature sensor, and an inertial sensor.

Preferably, the electronic module is offset with respect to the absorbent pad.

Preferably, the electronic module is located towards a periphery of the absorbent pad.

Preferably, the electronic module is located beyond the periphery of the absorbent pad.

Preferably, the electronic module is located beyond the periphery of the absorbent pad on a substrate.

Preferably, the electronic module comprises a communications module for communicating the wound data from the electronic module to a clinician, and wherein the communications module comprises a wireless communications module, and wherein the electronic module further comprises a power source for powering the electronic module.

Preferably, the dressing system further comprises a backing film on the absorbent pad.

The invention therefore results in a smart or intelligent wound dressing for the intelligent monitoring of wound health and dressing condition having integral moisture sensors adapted to map the progress of exudate in the dressing - i.e. the moisture sensors form part of the dressing (in particular the absorbent pad) or are located internally within the dressing The radially offset position of the (moisture) sensors can delay activation of the sensors by diffusing exudate until after about 50% of the dressing capacity is reached. In addition, by locating an array of the (moisture) sensors in a radial manner i.e. both centrally and offset from the centre of the absorbent towards the periphery of the dressing, exudate progress across the dressing can be measured or mapped to give a time-dependent indication of the saturation levels of the dressing.

Moreover, offset positioning of the electronic module ensures that dressing performance is not compromised by the presence of the electronic module. In particular, the offset electronic module does not impede or impinge upon exudate diffusion in the dressing. Although the Applicant does not wish to be bound by any theorem, it is believed that additional pressure created by centrally positioned electronic modules on the absorbent foam in previous dressings can alter the diffusive/wicking properties of the absorbent pad so that the dressing does not perform as intended. The offset position of the electronic module of the present invention eliminates any such additional pressures in the dressing and improves breathability.

The dressing of the invention is a laminated or multi-layer dressing adapted to rapidly absorb exudates and interstitial fluids and optimize conditions for healing at the wound-dressing interface. A primary wicking or absorbent pad provides a rapid capillary action response to quickly distribute absorbed exudate throughout the dressing and create a sustained movement of fluid away from wound beds.

The dressing of the invention facilitates the monitoring of individual and multiple sensor readings of a wound in real-time and/or the provision of time specific updates. The data generated from the dressing provides a constant and/or time specific update to an app and/or software available to a clinician. Each sensor reading provides a specific reading that is easily interpreted for ease of use and understanding.

In addition to the moisture sensors, pH sensors can be employed in the dressing system to facilitate the measurement of exudate pH to reflect the condition of a wound bed and aid in monitoring and determining the wound's response to treatment. Pressure sensors can also be employed to provide clinicians with key evidence describing pressure applied on wounds during critical healing stages. Temperature sensors can also be employed to monitor healing. Similarly, bacterial sensors can be used to assist in detecting infection. Inertial sensors can be adapted to monitor patient orientation, motion and/or activity.

The dressing of the invention provides a sealing arrangement preventing any bodily fluids or other material that may cause infection and/or impact sensor readings from reaching a wound. The waterproof sealing arrangement also enables the patient to wash and shower without damaging or obstructing the dressing. This is particularly beneficial when the dressing is employed on long term wounds.

Data generated from the dressing is used for the prediction of wound healing to help clinicians adopt a more specific management strategy, at the right time, to achieve healing. The use of the sensed parameters (e.g. moisture, pH and temperature) in combination provides essential information that improves future patient care.

Data transferred to a downloaded app on a handheld device can be used to provide clinicians with continuous data for monitoring and analysing wounds with the app enabling real-time bi-directional communication between patient and clinician.

### Brief Description of the Drawings

The invention will now be described, by way of example only, with reference to the accompanying drawings in which:
Figure 1 is a top plan view of a typical multi-layered intelligent wound dressing system for monitoring and treating a wound made up of an absorbent foam pad with a super-absorbent fibre (SAF) layer and a backing layer, and a moisture sensor centrally located in the dressing on top of the absorbent foam pad and under the backing film at the SAF layer;
Figure 2 is a cross-sectional view through the intelligent wound dressing of Figure 1 on a wound with the vertical and lateral direction of diffusion of wound exudate through the dressing indicated by the arrows;
Figure 3 is a top plan view of a first embodiment of a multi-layered intelligent wound dressing system of the invention for monitoring and treating a wound similar to the dressing system of Figure 1 in which an array of four integral moisture sensors on the absorbent pad in the dressing are radially offset from the centre of the absorbent pad and are located towards the periphery of the dressing at the corners of the absorbent pad;
Figure 4 is a top plan view of a second embodiment of a multi-layered intelligent wound dressing system of the invention for monitoring and treating a wound similar to the dressing system of Figure 3 in which a single moisture sensor is offset from the centre of the absorbent pad and is located towards the periphery of the dressing at a corner of the absorbent pad and a wicking/diffusion barrier in the form of a delay channel is provided in the dressing adjacent the moisture sensor to delay activation of the sensor;
Figure 5 is a cross-sectional view through a third embodiment of a multi-layered intelligent wound dressing system of the invention for monitoring and treating a wound similar to the dressing system of Figure 4 in which the moisture sensors are offset from the absorbent pad on a wicking/diffusion barrier in the form of a secondary super-absorbent fibre layer and the direction of diffusion of exudate is indicated by the arrows;
Figure 6 is a top plan view of a variant of the intelligent wound dressing system of Figure 3 with a radial spacing of moisture sensors and the electronic module is located beyond a periphery of the absorbent pad on a substrate;
Figure 7 is a graph of sensor performance of the moisture sensors in the intelligent dressings of Figures 1 to 5 showing the improved performance of the offset moisture sensors, and
Figure 8 is a graph of exudate threshold volume in the intelligent dressing system of Figure 6 according to sensor spacing in the dressing system.

### Detailed Description of the Invention

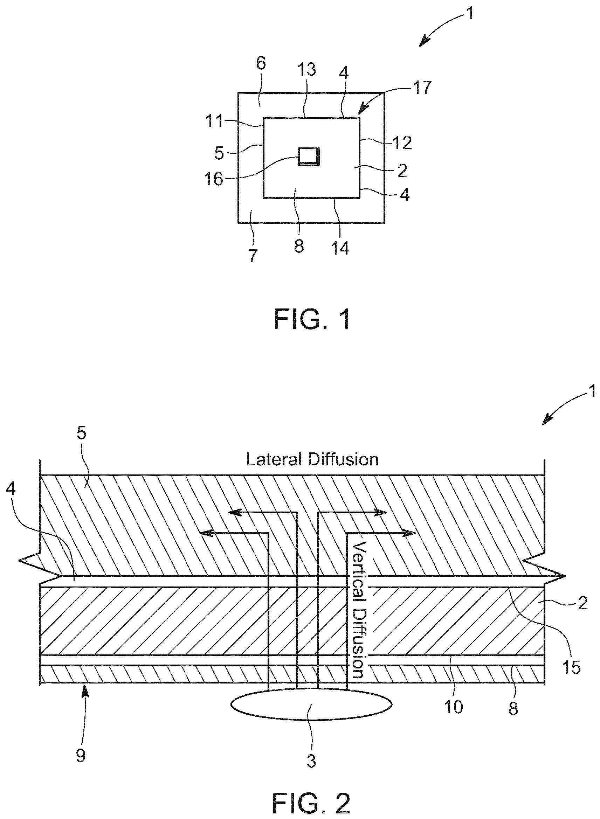

Figures 1 and 2 show a typical multi-layered intelligent wound dressing system for monitoring and treating a wound generally indicated by the reference numeral 1 and made up of a wicking absorbent foam pad 2 for absorbing wound exudate 3 having an intermediate non-absorbent layer 4 thereon and also an upper super-absorbent fibre (SAF) layer 5. The dressing system 1 also has an adhesive backing film 6 attached to the SAF layer 5 defining a peripheral adhesive side border 7 for adhering the dressing 1 to a patient. The absorbent pad 2 is provided with a silicon adhesive 8 for assisting in adhesion and a removable release film 9 on the absorbent pad 2 and the backing film 6 for protecting the dressing 1 prior to use.

The absorbent pad 2 is substantially square in shape and is made up of a substantially square lower wound face 10 for contacting a wound having four side walls 11,12,13,14 upstanding therefrom and an upper face 15 disposed towards the non-absorbent layer 4 and the SAF layer 5.

The dressing system 1 has a (moisture) sensor 16 centrally positioned with respect to the sidewalls 11,12,13,14 on top of the absorbent foam pad 2 and under the backing film 6 at the SAF layer 5. Accordingly, in practice, the centrally positioned moisture sensor 16 of the dressing system 1 is located directly above the exudate 3 source i.e. the wound. The dressing system 1 can also have additional moisture sensors 16 as required.

The dressing system 1 of Figures 1 and 2 is further provided with an electronic module 17 for harvesting and communicating data from the moisture sensor 10.

As shown in Figure 2, in use, diffusion of wound exudate 3 takes place initially by vertical diffusion from a wound, through the absorbent foam pad 2, the non-absorbent layer 4 and the SAF layer 5. At the SAF layer 5, fast lateral diffusion then takes place so that the surface of the SAF layer 5, which is in direct contact with the moisture sensor 16 becomes rapidly wet.

The fast lateral diffusion wicks moisture across the dressing system 1 very rapidly, and into contact with the moisture sensors which 16 which are then activated (i.e. show a sudden and significant drop in resistance where resistance moisture sensors 16 are employed) and saturate earlier than desired i.e. well before about 50% of the dressing capacity is reached. Alternatively any other type of sensor can be used that shows a change in measurand upon contact with or proximity to exudate.

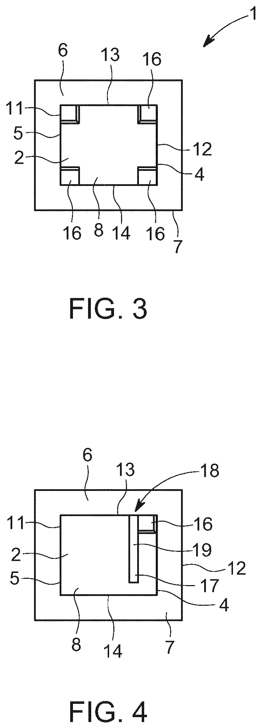

Figure 3 shows a top plan view of a first embodiment of a multi-layered intelligent wound dressing system 1 of the invention for monitoring and treating a wound similar to the dressing system 1 of Figure 1 and like numerals indicate like parts. However, the intelligent wound dressing system 1 of the invention is provided with an array of moisture sensors in the form of a plurality of moisture sensors 16 namely four integral moisture sensors 16 formed as part of the dressing system 1 positionally offset on and with respect to the centre of the absorbent pad 2 so that activation of the sensors 16 via the vertical and lateral diffusion of exudate 3 is delayed until after about 50% of the dressing capacity is reached - i.e. radial positional offsetting of the moisture sensors 16 causes a delay in diffusion of exudate 3 to the moisture sensors 16 so that travel of wound exudate 3 can be mapped and/or measured. More particularly, in the present embodiment, the four moisture sensors 16 are located towards the periphery of the intelligent dressing 1 e.g. towards the sidewalls 11,12,13,14 of the absorbent pad 2. More particularly, in the present embodiment, the four peripheral sensors 16 of the array are located in the four corners of the square absorbent pad 2.

Figure 4 shows a top plan view of a second embodiment of a multi-layered intelligent wound dressing system 1 of the invention similar to the dressing system of Figure 3 in which a single moisture sensor 16 is offset with respect to the centre of the absorbent pad 2 towards the periphery of the dressing 1. Like numerals indicate like parts.

More particularly, in the present embodiment, the moisture sensor 16 is located at the corner of the absorbent pad 2 defined between the sidewalls 12,13. However, the dressing system 1 is further provided with a diffusion/wicking barrier 18 to delay diffusion of exudate 3 towards the moisture sensor 16 to in turn to delay activation of the moisture sensor 16. In the present embodiment, the diffusion barrier 18 is a delay line or channel 19 defined in and across the absorbent foam pad 2 including the non-absorbent layer 4 and the SAF layer 5 adjacent the moisture sensor 16. The delay channel 19 is cut into absorbent foam pad 2, the non-absorbent layer 4 and the SAF layer 5 to partially diffusively isolate the moisture sensor 16 from the absorbent pad 2 (and hence the exudate 3) so that diffusion of exudate 3 to the moisture sensor 16 is delayed until after about 50% of the dressing capacity is reached. As the moisture sensor 16 is only partially diffusively isolated by the delay channel 19, the delay channel 19 extends only partially across the absorbent pad 2, the non-absorbent layer 4 and the SAF layer 5 so that the moisture sensor 16 remains diffusively communicable with exudate 3 in use.

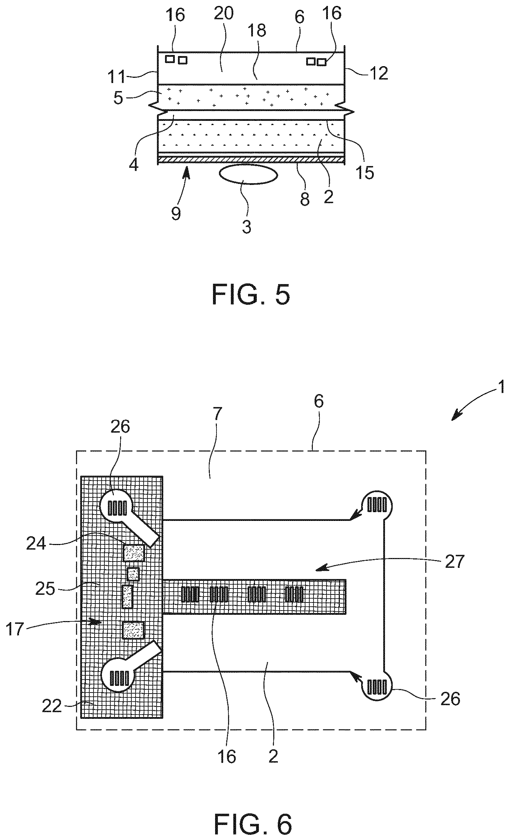

Figure 5 shows a cross-sectional view through a third embodiment of a multi-layered intelligent wound dressing system 1 of the invention similar to the dressing system 1 of Figure 4 but in which two moisture sensors 16 are offset with respect to the absorbent pad 2 on a diffusion barrier 18 in the form of a secondary superabsorbent (SAF) layer 20 i.e. a second superabsorbent layer 20 between the moisture sensors 16 and the first SAF layer 5. More particularly, the two moisture sensors 16 are offset with respect to the centre of the absorbent pad 2 towards the periphery of the dressing 1 on the secondary SAF layer 20 which is located between the (primary) SAF layer 5 and the backing film 6. The secondary SAF layer 20 is sized and dimensioned to delay exudate 3 diffusion so that sensor 16 activation is delayed until after about 50% of the dressing capacity is reached.

Figure 6 shows a top plan view of a variant of the intelligent wound dressing system 1 of Figure 3 with a radial spacing of moisture sensors 16 and like numerals indicate like parts. However, in the present embodiment, the electronic module 17 is also offset with respect to the centre of the absorbent pad 2 on a laminar substrate 22 to preserve and enhance the performance of the intelligent dressing 1. More particularly, in the present embodiment, the electronic module 17 is located to the left of the side wall 11 of the absorbent pad 2 on the substrate 22. The electronic module substrate 22 is accommodated in the multi-layer dressing system 1 and supports the electronic module 17 in the dressing system 1 fully offset from absorbent pad 2 so that the electronic module 17 does not overlie or overlay the absorbent pad 2. Accordingly, the electronic module 17 in the dressing system 1 does not impact, negatively or otherwise, on exudate 3 diffusion in the dressing system 1.

As shown in the drawing, in the present embodiment, the dressing system 1 is provided with radially offset peripheral moisture sensors 16 as previously described disposed towards the periphery of the dressing system 1. The offset moisture sensors 16 are positioned adjacent the corners of the side walls 11,12,13,14 of the absorbent pad 2 on peripheral tabs 26 connected to the absorbent pad 2. However, additionally, the dressing system 1 is provided with a further array 27 of moisture sensors 16. The moisture sensors 16 of the array 27 are radially offset from and/or spaced apart across the centre of the absorbent pad 2 between the side walls 11,12 towards the periphery of the dressing system 1 so that exudate 3 diffusion rates across the dressing system 1 can be measured by the array 27 of sensors 16.

The electronic module 17 on the substrate 22 can include a processor for processing data from the sensors 16, memory for storing data and programmes, a communications module 24 (e.g. a BLE, NFC, WiFi (Trade Mark) or narrowband IoT based communications module) for communicating the sensor data to a base station attended by a clinician, batteries 25 and an actuator for controlling actuation of the dressing system 1. The module can be made up of several sections electrically linked as appropriate. Other components can use an inertial sensor for patient activity monitoring, voltage regulators, switches, LED indicators etc. As shown in the drawing, all the aforementioned components can be accommodated on the electronic module substrate 22 in a fully offset position without overlying the absorbent pad 2.

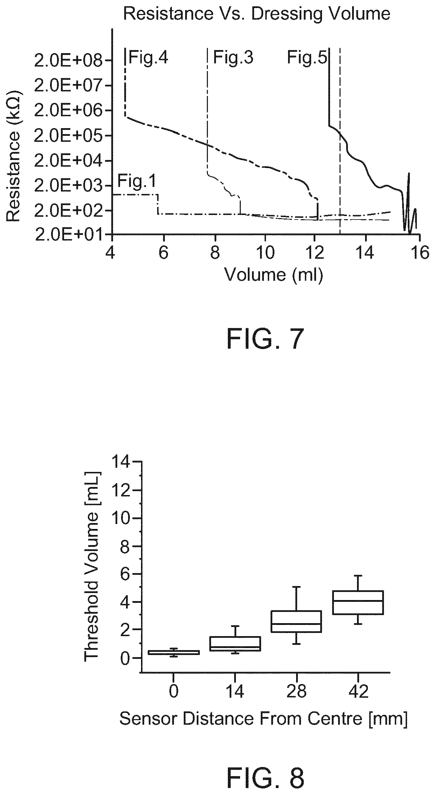

Figure 7 shows a graph of sensor performance of the moisture sensors in the intelligent dressings of Figures 1 to 5 showing the improved performance of the offset moisture sensors of the intelligent dressing systems 1 of Figures 3 to 5 when compared with that of the non-offset moisture sensor of the dressing system of Figure 1. The dressing systems 1 shown in Figure 8 were sized so that the dressing capacity was about 26mL i.e. the moisture sensors 16 were activated at about 13mL (50% of the dressing capacity). As shown in the graph, a progressive shift in sensor activation (a drop in resistance) occurred for the intelligent dressing systems 1 of Figures 3 to 5. Accordingly, by offsetting the moisture sensors away from the centre of the absorbent pad 2, both with and without the diffusion barrier 18 and the secondary SAF layer 20 the desired dressing saturation target was achieved.

Figure 8 shows a graph of exudate threshold volume in the intelligent dressing system 1 of Figure 6 according to the radial moisture sensor 16 spacing in the dressing system 1. More particularly, the graph illustrates the threshold volume (i.e. the amount of exudate required to activate the moisture sensors 16) as a function of sensor 16 distance from the centre of the dressing system 1. As shown in the graph, sensors 16 placed at or near the dressing centre activated early while those towards the periphery of the dressing system remained inactive until more exudate 3 was absorbed by the dressing system 1. Accordingly, wound exudation rates could be assessed, the diffusion of exudate across the dressing measured, and predictions made regarding the time at which the dressing would become fully saturated and require changing.

The absorbent pad 2 is formed from a die-cut adhesive bandage material which allows for sufficient transfer of oxygen to a wound site while effectively preventing passage of microbes to the wound. The backing film 6 can be formed from a polymer such as acrylic or polyurethane while the release film 9 can be formed from polyethylene terephthalate (PET).

The electronic module 17 of the dressing system of the invention includes a communications module such as a wireless communications module for communicating wound data from the electronic module to a clinician and a power source such as a battery for powering the electronic module.

The dressing system 1 can be manufactured in any suitable size as required in accordance with wound sizes. Typical dressing sizes are 10cm x 10cm, 7.5cm x 7.5cm, 20cm x 10cm and 20cm x 20cm.

The dressing system 1 is sterilised to kill microorganisms transferred during the manufacturing process. A suitable sterilization method is an ethylene oxide (EtO) sterilisation method which protects the sensors 16 and electronic module 17 from damage. This method of sterilisation is also preferred due to its handling ease, versatility and suitability for use with delicate medical dressings which could be damaged by other sterilisation methods such as heat sterilisation.

In use, data from the sensors 16 is harvested and processed for optimal wound monitoring and healing. Other sensor types can be employed in the dressing system 1 in addition to the moisture sensors 16 as required e.g. pressure sensors, bacterial sensors, temperature sensors and pH sensors. The sensors 16 can be offset from the centre of the absorbent pad 16 as hereinbefore described and can also be included in the offset electronic module 17 if desired. Various data processing methods can be employed as necessary.

The invention is not limited to the embodiments herein described which may be varied in construction and detail without departing from the scope of the invention.

## Claims

1. A dressing system (1) for a wound comprising:
an absorbent pad (2);
at least one sensor (16) for detecting wound data, and
an electronic module (17) communicable with the sensor, **characterised in that**:
the sensor (16) comprises a moisture sensor array (27) provided on or in the absorbent pad (2) adapted to map the progress of exudate (3) in the absorbent pad (2), and wherein the moisture sensor array comprises a plurality of moisture sensors (16) radially offset with respect to the centre of the absorbent pad (2) including a peripheral moisture sensor (16) located towards a periphery of the absorbent pad.

2. A dressing system (1) as claimed in Claim 1 wherein the peripheral sensor (16) is located beyond the periphery of the absorbent pad.

3. A dressing system (1) as claimed in Claim 1 or Claim 2 wherein the dressing system further comprises a diffusion barrier (18) to delay diffusion of wound exudate (3) towards the sensor (16).

4. A dressing system (1) as claimed in Claim 3 wherein the diffusion barrier (18) comprises a delay channel (19) defined in the absorbent pad (2).

5. A dressing system (1) as claimed in Claim 3 wherein the absorbent pad (2) comprises a first superabsorbent layer (5) and the diffusion barrier (18) comprises a second superabsorbent layer (20) between the sensor (16) and the first superabsorbent layer (5), and wherein the first (5) and second (20) superabsorbent layers comprise superabsorbent fibre layers.

6. A dressing system (1) as claimed in any of Claims 1 to 5 wherein the moisture sensors (16) of the moisture sensor array (27) are radially offset with respect to the centre of the absorbent pad (2), to delay activation of the moisture sensors (16) by diffusing exudate until after about 50% of the dressing system capacity is reached.

7. A dressing system (1) as claimed in any of Claims 1 to 6 wherein exudate (3) progress across the dressing is measurable to provide a time-dependent indication of saturation levels of the dressing.

8. A dressing system (1) as claimed in any of Claims 1 to 7 wherein the sensor further comprises one of: a pH sensor, a pressure sensor, a bacterial sensor, a temperature sensor, and an inertial sensor.

9. A dressing system (1) as claimed in any of Claims 1 to 8 wherein the electronic module (17) is offset with respect to the absorbent pad.

10. A dressing system (1) as claimed in Claim 9 wherein the electronic module (17) is located towards a periphery of the absorbent pad.

11. A dressing system (1) as claimed in Claim 10 wherein the electronic module (17) is located beyond the periphery of the absorbent pad (2).

12. A dressing system (1) as claimed in Claim 11 wherein the electronic module (17) is located beyond the periphery of the absorbent pad (2) on a substrate (22).

13. A dressing system (1) as claimed in any of Claims 1 to 12 wherein the electronic module (17) comprises a communications module for communicating the wound data from the electronic module (17) to a clinician, and wherein the communications module comprises a wireless communications module, and wherein the electronic module (17) further comprises a power source for powering the electronic module.

14. A dressing system (1) as claimed in any of Claims 1 to 13 wherein the dressing system further comprises a backing film (6) on the absorbent pad (2).

## Patentansprüche

1. Verbandsystem (1) für eine Wunde, umfassend:
ein absorbierendes Kissen (2);
mindestens einen Sensor (16) zum Erkennen von Wunddaten, und
ein elektronisches Modul (17), das mit dem Sensor kommunizieren kann,
**dadurch gekennzeichnet, dass**:
der Sensor (16) eine Feuchtigkeitssensoranordnung (27) umfasst, die auf oder in dem absorbierenden Kissen (2) bereitgestellt ist und geeignet ist, den Verlauf des Exsudats (3) in dem absorbierenden Kissen (2) abzubilden, und wobei die Feuchtigkeitssensoranordnung eine Vielzahl von Feuchtigkeitssensoren (16) umfasst, die in Bezug auf die Mitte des absorbierenden Kissens (2) radial versetzt sind und einen peripheren Feuchtigkeitssensor (16) einschließen.

2. Verbandsystem (1) nach Anspruch 1, wobei der periphere Sensor (16) außerhalb des Umfangs des absorbierenden Kissens angeordnet ist.

3. Verbandsystem (1) nach Anspruch 1 oder 2, wobei das Verbandsystem ferner eine Diffusionsbarriere (18) umfasst, um die Diffusion von Wundexsudat (3) in Richtung des Sensors (16) zu verzögern.

4. Verbandsystem (1) nach Anspruch 3, wobei die Diffusionsbarriere (18) einen Verzögerungskanal (19) umfasst, der in dem absorbierenden Kissen (2) definiert ist.

5. Verbandsystem (1) nach Anspruch 3, wobei das absorbierende Kissen (2) eine erste superabsorbierende Schicht (5) umfasst und die Diffusionsbarriere (18) eine zweite superabsorbierende Schicht (20) zwischen dem Sensor (16) und der ersten superabsorbierenden Schicht (5) umfasst, und wobei die erste (5) und zweite (20) superabsorbierende Schicht superabsorbierende Faserschichten umfassen.

6. Verbandsystem (1) nach einem der Ansprüche 1 bis 5, wobei die Feuchtigkeitssensoren (16) der Feuchtigkeitssensoranordnung (27) in Bezug auf die Mitte des absorbierenden Kissens (2) radial versetzt sind, um die Aktivierung der Feuchtigkeitssensoren (16) durch diffundierendes Exsudat zu verzögern, bis etwa 50 % der Kapazität des Verbandsystems erreicht sind.

7. Verbandsystem (1) nach einem der Ansprüche 1 bis 6, wobei das Fortschreiten des Exsudats (3) über den Verband messbar ist, um eine zeitabhängige Anzeige des Sättigungsgrades des Verbandes zu liefern.

8. Verbandsystem (1) nach einem der Ansprüche 1 bis 7, wobei der Sensor ferner einen von: einem pH-Sensor, einem Drucksensor, einem bakteriellen Sensor, einem Temperatursensor und einem Inertialsensor umfasst.

9. Verbandsystem (1) nach einem der Ansprüche 1 bis 8, wobei das elektronische Modul (17) in Bezug auf das absorbierende Kissen versetzt ist.

10. Verbandsystem (1) nach Anspruch 9, wobei das elektronische Modul (17) in Richtung eines Umfangs des absorbierenden Kissens angeordnet ist.

11. Verbandsystem (1) nach Anspruch 10, wobei das elektronische Modul (17) außerhalb des Umfangs des absorbierenden Kissens (2) angeordnet ist.

12. Verbandsystem (1) nach Anspruch 11, wobei das elektronische Modul (17) außerhalb des Umfangs des absorbierenden Kissens (2) auf einem Substrat (22) angeordnet ist.

13. Verbandsystem (1) nach einem der Ansprüche 1 bis 12, wobei das elektronische Modul (17) ein Kommunikationsmodul zum Kommunizieren der Wunddaten von dem elektronischen Modul (17) an einen Kliniker umfasst, und wobei das Kommunikationsmodul ein drahtloses Kommunikationsmodul umfasst, und wobei das elektronische Modul (17) ferner eine Energiequelle zum Versorgen des elektronischen Moduls umfasst.

14. Verbandsystem (1) nach einem der Ansprüche 1 bis 13, wobei das Verbandsystem ferner eine Trägerfolie (6) auf dem absorbierenden Kissen (2) umfasst.

## Revendications

1. Système de pansement (1) pour une plaie, comprenant :
une compresse absorbante (2) :
au moins un capteur (16) destiné à détecter les données de plaie, et
un module électronique (17) pouvant communiquer avec le capteur, **caractérisé en ce que** :
le capteur (16) comprend un réseau de capteur d'humidité (27) prévu sur la compresse absorbante (2) ou dans celle-ci et adapté pour cartographier la progression de l'exsudat (3) dans la compresse absorbante (2), et dans lequel le réseau de capteur d'humidité comprend une pluralité de capteurs d'humidité (16) décalés radialement par rapport au centre de la compresse absorbante (2), incluant un capteur d'humidité périphérique (16) situé vers une périphérie de la compresse absorbante.

2. Système de pansement (1) selon la revendication 1, dans lequel le capteur périphérique (16) est situé au-delà de la périphérie de la compresse absorbante.

3. Système de pansement (1) selon la revendication 1 ou la revendication 2, dans lequel le système de pansement comprend en outre une barrière de diffusion (18) destinée à retarder une diffusion d'un exsudat de plaie (3) vers le capteur (16).

4. Système de pansement (1) selon la revendication 3, dans lequel la barrière de diffusion (18) comprend un canal de retard (19) défini dans la compresse absorbante (2).

5. Système de pansement (1) selon la revendication 3, dans lequel la compresse absorbante (2) comprend une première couche superabsorbante (5) et la barrière de diffusion (18) comprend une seconde couche superabsorbante (20) entre le capteur (16) et la première couche superabsorbante (5), et dans lequel les première (5) et seconde (20) couches superabsorbantes comprennent des couches de fibre superabsorbantes.

6. Système de pansement (1) selon l'une quelconque des revendications 1 à 5, dans lequel les capteurs d'humidité (16) du réseau de capteur d'humidité (27) sont décalés radialement par rapport au centre de la compresse absorbante (2), afin de retarder l'activation des capteurs d'humidité (16) par diffusion de l'exsudat jusqu'à ce que environ 50 % de la capacité de système de pansement soit atteinte.

7. Système de pansement (1) selon l'une quelconque des revendications 1 à 6, dans lequel la progression de l'exsudat (3) à travers le pansement est mesurable afin de fournir une indication dépendante du temps des niveaux de saturation du pansement.

8. Système de pansement (1) selon l'une quelconque des revendications 1 à 7, dans lequel le capteur comprend en outre un élément parmi : un capteur de pH, un capteur de pression, un capteur bactérien, un capteur de température et un capteur inertiel.

9. Système de pansement (1) selon l'une quelconque des revendications 1 à 8, dans lequel le module électronique (17) est décalé par rapport à la compresse absorbante.

10. Système de pansement (1) selon la revendication 9, dans lequel le module électronique (17) est situé vers une périphérie de la compresse absorbante.

11. Système de pansement (1) selon la revendication 10, dans lequel le module électronique (17) est situé au-delà de la périphérie de la compresse absorbante (2).

12. Système de pansement (1) selon la revendication 11, dans lequel le module électronique (17) est situé au-delà de la périphérie de la compresse absorbante (2) sur un substrat (22).

13. Système de pansement (1) selon l'une quelconque des revendications 1 à 12, dans lequel le module électronique (17) comprend un module de communication permettant de communiquer les données de plaie à partir du module électronique (17) à un clinicien, et dans lequel le module de communication comprend un module de communication sans fil, et dans lequel le module électronique (17) comprend en outre une source de puissance pour alimenter en puissance le module électronique.

14. Système de pansement (1) selon l'une quelconque des revendications 1 à 13, dans lequel le système de pansement comprend en outre un film de support (6) sur la compresse absorbante (2).
